# EUROPÄISCHE PATENTANMELDUNG

(11) **EP 0 878 703 A2**
(43) Veröffentlichungstag der Anmeldung: **18.11.1998**
(21) Anmeldenummer: 98105560.1
(22) Anmeldetag: 26.03.1998
(51) Int. Cl.: G01N 21/35, G01N 21/53, G01N 25/18, G01N 25/20

(54) **Kombinierter Gassensor zur Detektion von Gasen und Partikeln mit Betriebsverfahren und Verwendungen**

(30) Priorität: 13.05.1997 DE 19720007
(71) Anmelder: SIEMENS AKTIENGESELLSCHAFT, 80333 München (DE)
(72) Erfinder: Fleischer, Maximilian, Dr., 85635 Höhenkirchen (DE); Freitag, Reinhard, 81379 München (DE); Meixner, Hans, Prof., 85540 Haar (DE); Frank, Joachim, 85521 Ottobrunn (DE)

(57) **Zusammenfassung**

Es wird ein integrierter Aufbau eines Gassensors bestehend aus einem beheizten Gassensor und einem Gassensor nach dem Infrarotabsorptionsprinzip beschrieben. Der beheizte Gassensor, beispielsweise ein Metalloxid-Halbleitersensor dient gleichzeitig als Infrarotstrahlungsquelle für den Absorptionsgassensor. Die den Gassensoren zugrundeliegenden Detektionsprinzipien können gleichzeitig für ein oder mehrere Gase verwendet werden. Zusätzlich kann durch die Messung der Lichtstreuung an vorhandenen Partikeln die Detektion von offenen Bränden betrieben werden.

## Beschreibung

Die Erfindung betrifft die multifunktionale Gasdetektion mit einem Sensor unter Einsatz von Sensorsystemen, die einerseits selektiv anhand der Infrarot-Absorption detektieren und andererseits breitbandig aufgrund von gaskonzentrationsabhängigen Materialkennwerten detektieren.

Bisherige Gassensoren detektieren entweder Gase selektiv, wie beispielsweise durch die Infrarot-Absorption, oder breitbandig, wie beispielsweise mittels eines beheizten Metalloxid-Gassensors. Für die Aufgaben der Raumluftüberwachung ist beispielsweise eine Kombination aus selektiver und breitbandiger Gasdetektion wünschenswert.

Beispielsweise besteht ein großer Bedarf an Sensoren zur Detektion von CO₂ zum Einsatz bei der Lüftungs- und Klimasteuerung in Wohn- und Arbeitsräumen, sowie in Keller- und Lagerräumen. Außerdem soll in den genannten Räumen zusätzlich auch das Auftreten von verschiedenen anderen beispielsweise explosiven und auch von geruchsbelästigenden Gasen detektiert werden. Hier sind beispielsweise Erd- und Flüssiggas, bzw. Zigarettenrauch, TVOC - Total Volatile Organic Compounds - zu nennen.

Verschiedene Sensoren zeigen trotz bestehender Verunreinigung ein Signal, welches aufgrund einer Fehlfunktion einen fehlerhaften Meßwert darstellt. Zum Schutz vor Stauben werden z.B. Fließstoff-Filter eingesetzt. Hierbei ist zu beachten, daß mit zunehmender Dichte des Filters auch die Gasaustauchzeiten ansteigen, was nur in gewissen Grenzen in Kauf genommen werden kann. Bei anderen Anordnungen wird eine Verschmutzung des optischen Fensters in Kauf genommen. Dann wird durch eine intelligente und aufwendigere Auswerteelektronik als üblich, die Änderung durch eine Konzentrationsänderung des Zielgases von der vergleichsweise langsamen Drift des Sensorsignals durch Verschmutzungen des optischen Fensters separiert. Dieses elektronisch aufwendigere Verfahren versagt jedoch dann, wenn sich die Konzentration des Zielgases in dem wichtigen Konzentrationsbereich eben nicht schnell ändert.

Kommerziell erhältliche CO₂-Sensoren nutzen meist optische Effekte, insbesondere die Infrarot-Absorption. Andere Meßverfahren sind entweder prinzipiell nicht anwendbar, da stabile CO₂-Moleküle nicht mit Festkörpern bzw. mit deren Oberflächen reagieren, was z.B. eine Detektion mit Gassensoren auf der Basis halbleitender Metalloxide ausschließt, oder sie leiden unter mangelnder Selektivität. Dies gilt beispielsweise bei der Detektion von CO₂ aufgrund einer veränderten Wärmeleitfähigkeit des Gasgemisches als Messgröße.

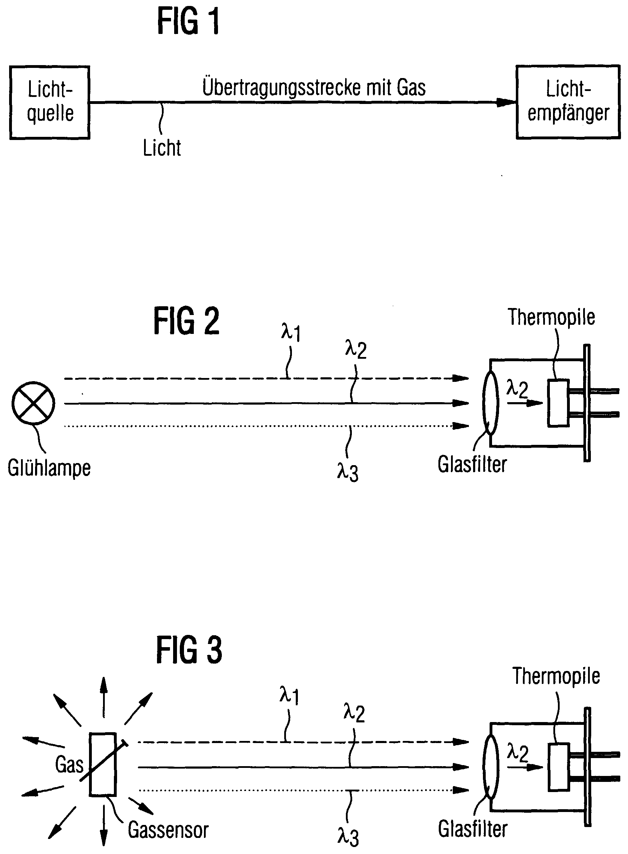

Bei der optischen Detektion mit der Infrarot-Absorptions-Analyse wird die Tatsache ausgenutzt, daß bei Anwesenheit eines mehratomigen Zielgases ein bestimmter Spektralbereich im Infrarotbereich des Lichtes deutliche gedämpft wird. Ein hierzu verwendeter Aufbau für die optische Detektion von Gasen besteht prinzipiell aus einer Strahlungsquelle, einer Übertragungsstrecke oder Absorptionsstrecke, sowie einem Strahlungsempfänger. Innerhalb der Absorptionsstrecke werden bestimmte Wellenlängen der Strahlung von dem Gas absorbiert. Die Gasselektivität eines solchen optischen Sensors wird dadurch erreicht, daß entweder die Strahlungsquelle nur in dem Wellenlängenbereich abstrahlt, der von dem zu detektierenden Gas absorbiert wird (z.B. Laserdiode) oder daß in der Absorptionsstrecke ein Transmissionsfilter vorhanden ist, daß nur in dem Wellenlängenbereich durchlässig ist, der von dem zu detektierenden Gas absorbiert wird (z.B. beschichtete Glasfenster bei Thermopiles, Interferenzfilter). In Figur 1 wird der entsprechende Sachverhalt verdeutlicht.

In Zusammenhang mit der Darstellung in Figur 2 wird gezeigt, daß eine typische gebräuchliche Ausführung eines beschriebenen Sensors eine Glühlampe als breitbandige Strahlungsquelle verwendet und ein Thermopile (Thermosäule) als Strahlungsempfänger, wobei im Gehäuse des Thermopiles, bei direkter Einstrahlung mit der Strahlungsquelle zugewandter Anordnung, ein wellenlängenselektives Glasfilter eingesetzt wird. In diesem Aufbau dient die Wegstrecke zwischen Glühlampe und Thermopile als Absorptionsstrecke. Das wellenlängenselektive Glasfilter hat für die CO₂-Detektion beispielsweise einen Transmissionsbereich für die Wellenlangen von 4,2 bis 4,4 µm. In diesem Bereich liegt eine Vielzahl starker Absorptionslinien von CO₂.

Besteht außer der CO₂-Detektion zusätzlich die Anforderung zum Nachweis anderer Gase, wie beispielsweise der Nachweis toxischer oder explosiver Gase, so ist die Verwendung eines weiteren Gassensors notwendig. Bestehen geringe Anforderungen, z.B. hinsichtlich der Selektivität, so ist die Verwendung von Gassensoren auf der Basis halbleitender Metalloxide für die Detektion weiterer Gase möglich. Durch die Verwendung eines weiteren Gassensors steigen allerdings auch die Kosten sowie die Gesamtleistungsaufnahme der Anordnung. Beides sind wesentliche Faktoren für den Einsatz von Gassensoren in dem potentiellen Massenmarkt der Domotik und der Gebäudetechnik, deren Höhe ausschlaggebend ist für die Akzeptanz der Gassensoren.

Weiterhin ist eine frühzeitige und sichere Branderkennung in Wohngebäuden, öffentlichen Gebäuden und industriellen geschlossenen Anlagen durch einen kostengünstigen Aufbau von Sensoren erwünscht. Die frühzeitige Branderkennung in Gebäuden stellt eine wesentliche Maßnahme zur Vermeidung von Schäden an Personen und Beschädigungen von Sachwerten dar. Neben dem Auslösten optischer und akustischer Warnungen werden im Zuge neuerer Entwicklungen, wie dem Konzept des "intelligenten Hauses" weitere automatisierte Reaktionsmöglichkeiten diskutiert. Bei dem genannten Konzept sind vielfältige Sensoren und Aktoren miteinander vernetzt. Die Branderkennung durch ein entsprechendes Sensorsystem löst dann neben der Warnung weitere Aktionen, wie z.B. Notrufeinleitung bei der Feuerwehr, Schließen des Fensters bei Abwesenheit von Personen, Öffnen der Fenster bei Anwesenheit von Personen, Abschalten der Gasversorgung oder ggf. Auslösung eines Feuerlöschsystemes, aus. Im industriellen Bereich stellt die fehlalarmsichere Branddetektion einen Kostenfaktor dar, da durch häufige Fehlalarme unnötige und kostenintensive Abläufe ausgelöst werden.

Bisherige Brandmeldesysteme basieren im wesentlichen auf dem Prinzip von :
- Ionisationsmeldern mit einem kleinen radioaktiven Präparat in der Mitte eines Detektionsvolumens von Luft,
- Rauchmeldern, wobei die durch Rauch verursachte Trübung in der Luft durch Messung der Lichtabsorption detektiert wird,
- Temperaturfühlern, die abnormal hohe Temperaturen detektieren, und
- unselektiven Gassensoren bzw. Gassensorsystemen.

Die genannten Brandmeldesysteme aus dem Stand der Technik weisen jedoch verschiedene Nachteile auf. Das erstgenannte System benötigt ein radioaktives Präparat und findet daher besonders im privaten, aber auch zunehmend im industriellen Bereich geringe Akzeptanz. Der Rauchmelder gewährleistet nicht die sichere und schnelle Detektion aller Brandarten. Temperaturfühler erfüllen keinesfalls das Kriterium der frühzeitigen Branderkennung. Der letztgenannte Vorschlag, die Verwendung unselektiver Gassensoren (z.B. auf der Basis halbleitender Metalloxide) hat sich in der Praxis als bisher nicht verwendbar gezeigt. Die typischen halbleitenden Metalloxidsensoren (wie beispielsweise SnO₂, ZnO, Ga₂O₃, SrTiO₃, ...) reagieren auf eine Vielzahl reduzierender Gase. So reagieren diese nicht nur auf brandbegleitende Gase wie Kohlenmonoxid, Wasserstoff und verbrannte Kohlenwasserstoffe, sondern genauso auf Lösungsmittel, wie beispielsweise Alkohol oder Aceton oder sogar auf organische riechende Stoffe. Dies führt zur häufigen Auslösung von Fehlalarmen. Mehrsensorsysteme mit aufwendiger Datenauswertung vermögen Fehlalarme zu vermeiden. Diese Mehrsensorsysteme sind jedoch viel zu teuer und weisen Instabilitäten im Langzeitverhalten auf. Verbesserte selektive Gassensoren, z.B. selektive Wasserstoffsensoren, könnten hinsichtlich der Sicherheit auf Fehlalarm und hinsichtlich der Kostengünstigkeit deutlich Vorteile erbringen, was die verdeckten Brände (Schwelbrand und Brände unter Sauerstoffmangel) betrifft. Der Nachteil, daß sie eine recht geringe Empfindlichkeit auf offene Brände bei Sauerstoffüberschuß aufweisen, beruht darauf, daß es in diesem Fall nicht zu einer starken Emission unverbrannter Gase kommt, wie beispielsweise Wasserstoff und Kohlenwasserstoffe, auf die diese Sensoren reagieren.

Der Erfindung liegt die Aufgabe zugrunde, ein Gassensorsystem bzw. ein Gasdetektionsverfahren zur Verfügung zu stellen, womit toxische oder explosive Gase gleichzeitig neben geruchsbelästigenden Gasen detektierbar sind. Zusätzlich soll die Detektion von Schwelbränden und von offenen Bränden ermöglicht werden.

Die Lösung dieser Aufgabe geschieht durch die Merkmale von Anspruch 1 bzw. Anspruch 13 oder Anspruch 18.

Vorteilhafte Ausgestaltungen sind den Unteransprüchen zu entnehmen.

Der Erfindung liegt die Erkenntnis zugrunde, daß mittels einer multifunktionalen Gasdetektion mit der Kombination von beheizten Gassensoren, insbesondere Hochtemperatur-Gassensoren, und optischer Absorption ein preiswertes multifunktionales Sensorsystem erkannt wurde, das besonders für den Einsatz in der Raumluftüberwachung geeignet ist. Dieses Sensorsystem nutzt einerseits die Infrarot-Absorption, z.B. zur Detektion von CO₂ in der Luft, andererseits aber auch die gassensitiven Eigenschaften von beheizten Gassensoren,insbesondere die elektrischen Eigenschaften von Metalloxiden als Sensorelement. Diese bei hohen Temperaturen (400 bis 900°C) betriebenen Sensoren dienen als Quelle der Infrarotstrahlung. Die emittierte Infrarotstrahlung durchläuft auf dem Weg zum Infrarotdetektor (z.B. ein Thermopile) eine gasgefüllte Kammer, in der sie u.a. von dem Zielgas, z.B. CO₂, wellenlängenselektiv absorbiert wird. Eine Gassensitivität dieses Infrarotsensors wird dadurch erreicht, daß vor dem Detektor ein Infrarot-Bandpaßfilter eingesetzt wird, das nur Strahlung an der jeweiligen Absorptionsbande des Zielgases durchlaßt Bei CO₂ ist dies der Bereich von 4,2 bis 4,4 µm. Bei Anwesenheit des Zielgases verringert sich die durch das Filter auf den Detektor gelangende Strahlungsintensität merklich. Als Meßsignal dient bei der Verwendung eines Thermopiles die Änderung von dessen Ausgangsspannung.

Durch die Verwendung eines erfindungsgemäßen Sensoraufbaues kann eine Verschmutzung des optischen Fenster, beispielsweise vermieden werden. Außerdem wird durch den beschriebenen Aufbau ein rauschfreies und stabiles Meßsignal erzielt. Die Vorteile sind darauf begründet, daß ein solches Meßsignal auch hervorgerufen wird, wenn sich Verunreinigungen auf dem Gehäuses des Thermopiles ablagern und ein optisches Fenster bedecken, da diese Ablagerungen ebenfalls Infrarotstrahlung absorbieren. Das Sensorsystem würde in diesem Fall eine Fehlfunktion aufweisen. Bei Einsatz eines auf 900°C beheizten Sensors, beispielsweise eines Metalloxidsensors, bleiben derartige Verschmutzungen aus, da in diesem Fall organische Verunreinigungen bei derartig hohen Temperaturen verbrannt werden.

Hochtemperaturgassensoren auf der Basis halbleitender Metalloxide werden typischerweise im Temperaturbereich von 400 bis 1000°C betrieben. Somit können diese Hochtemperaturgassensoren einfach als Quelle für eine Infrarotstrahlung anstelle einer Glühlampe oder einer anderen separaten Lichtquelle verwendet werden. Diese Strahlungsquelle hat somit eine Doppelfunktion, die aus der eigenen Gasdetektion sowie der Bereitstellung der Infrarotstrahlung für einen Absorptionssensor innerhalb des Kombisensors besteht. Bei den genannten Temperaturen wird ein großer Teil der benötigten Heizleistung in Form von Strahlung abgegeben. Ein Großteil dieser Strahlung entfällt wiederum auf den für die Detektion mehratomiger Gase interessanten Wellenlängenbereich von 1 bis 10 µm. Die hohe Betriebstemperatur des Hochtemperaturgassensors von 400 bis 1000°C bewirkt in diesem Bereich eine hohe spektrale Strahlungsdichte. Somit liegt eine hinreichend starke Infrarotlichtquelle vor, mit der die Glühlampe nach dem Stand der Technik substituiert werden kann. Die Funktion des optischen Detektors, beispielsweise für CO 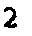 (Absorptionssensor), ist somit die gleiche wie oben allgemein beschrieben. Neben der möglichen selektiven Detektion von CO₂ ist mit der gleichen Anordnung ohne weiteren Aufwand zusätzlich auch die Detektion weiterer Gase möglich, z.B. mit einem bei 800°C betriebenen Ga₂O₃-Gassensor, nämlich die Detektion von explosiven Gasen, wie Methan oder Propan (Flüssiggas).

Eine vorteilhafte Ausgestaltung der Erfindung sieht den Einbau des Sensorsystemes in einem Gehäuse vor, z.B. in einem senkrecht stehenden Rohr, vorzugsweise aus Aluminium. Weitere vorteilhafte Möglichkeiten sind polierter Edelstahl oder Rohre, die auf der Innenseite mit edlen Metallen, wie beispielsweise Gold, Platin, Chrom oder Nickel, beschichtet sind. Kennzeichen der inneren Wandung des Rohres ist eine möglichst hohe Reflektivität im Verwendeten Infrarotspektralbereich und ausreichende Korrosionsbeständigkeit. Beide Enden des Rohres werden offengehalten und lediglich mit einem mehr oder weniger engmaschigen Netz, vorzugsweise aus Metall, versehen. Dieses verhindert das Eindringen großer Partikel, wobei diese Funktion vor allem von dem Netz am oberen Ende wahrgenommen wird, da hier aufgrund der Schwerkraft die Tendenz am größten ist, dass Partikel in die Sensoreinheit hineinfallen. Beide Netze vermeiden gleichzeitig Störungen des Sensorsystemes, die durch eindringende Verwirbelungen der umgebenden Luft hervorgerufen werden können. Alternativ denkbar sind wabenförmige Metallgebilde, die eine möglichst hohe Zahl durchgehender Kanäle aufweisen. Diese Strukturen bewirken eine nochmals verbesserte Unempfindlichkeit des Aufbaues gegenüber äußeren Luftverwirbelungen.

Der beheizte Gassensor, vorzugsweise ein geheizter Metalloxidsensor, dient auch in dieser Anordnung als Infrarot-Strahlungsquelle und das Thermopile als Infrarot-Detektor. Beide werden in ein senkrecht aufgehängtes Rohr integriert, so daß sich das Thermopile oberhalb der Infrarot-Strahlungsquelle befindet. Die nach außen zeigende Fläche des Thermopiles ist derart angeordnet, daß sie in Richtung auf das im Gehäuse integrierte Filter senkrecht nach unten zeigt. Die in die Meßkammer eindringende Luft wird durch den heißen Metalloxidgassensor erwärmt, erfährt eine Verringerung der spezifischen Dichte und steigt in dem Rohr aufgrund des sich ausbildenden Kamineffektes nach oben. Durch diesen sich ausbildenden Luftstrom ist es möglich, die Maschenweite der Metallnetze zu verringern, ohne daß sich lange Gasaustauschzeiten einstellen, wie sie in dem Fall vorliegen, wenn der Kamineffekt nicht genutzt wird. Der Kamineffekt wird nicht genutzt, wenn die Anordnung des Meßrohres waagerecht ist oder wenn Infrarot-Strahlungsquellen mit geringer Wärmeenergie eingesetzt werden. Durch den konstanten Luftstrom bei einer erfindungsgemäßen Anordnung verringert sich außerdem die Wahrscheinlichkeit, daß sich kleine Partikel an den Innenwänden des Meßrohres ablagern und dessen Reflexionsvermögen verringern. Die Erzielung besonderer Vorteile geschieht jedoch dadurch, dass die Anordnung verhindert, daß auf dem nach unten gerichteten optischen Filter sich aufgrund der entgegenwirkenden Schwerkraft Partikel absetzen können. Da diese Partikel sich auch nicht auf den heißen Metalloxid-Gassensor absetzen können, ist eine Verschmutzung aller optischen wirksamen Teile, also auch des optischen Sensors der eine Absorptionsmessung durchführt, weitestgehend ausgeschlossen. Somit ist das Problem der Sauberhaltung des für optische Gassensorik benötigten optischen Fensters gelöst.

Durch eine Begrenzung des Einflusses äußerer Luftverwirbelungen sowie den durch den Kamineffekt beruhigteren Gasstrom durch die Meßzelle wird ein rauschfreieres und kurzzeitig stabileres Meßsignal erreicht.

Im folgenden werden anhand der schematischen und den Patentgegenstand nicht einschränkenden Figuren Ausführungsbeispiele beschrieben:
- Figur 1: zeigt eine schematische Darstellung des prinzipiellen Aufbaues eines optischen Gasdetektors,
- Figur 2: zeigt den prinzipiellen Aufbau eines optischen Gasdetektors mit einer Glühlampe und einem Thermopile,
- Figur 3: zeigt den prinzipiellen Aufbau eines optischen Gasdetektors mit Gassensor als Strahlungsquelle und einem Thermopile,
- Figur 4: zeigt die hohe spektrale Strahlungsdichte in Abhängigkeit von der Wellenlänge,
- Figur 5: zeigt Verläufe der temperaturabhängigen Gassensitivitäten für verschiedene Gase eines Leitfähigkeitssensors (geheizter Sensor) auf der Basis des halbleitendem GA₂O₃,
- Figur 6a und 6b: zeigen mögliche Vorrichtungen zur Verlängerung der Übertragungs- bzw. Absorptionsstrecke, um die Absorption zu erhöhen,

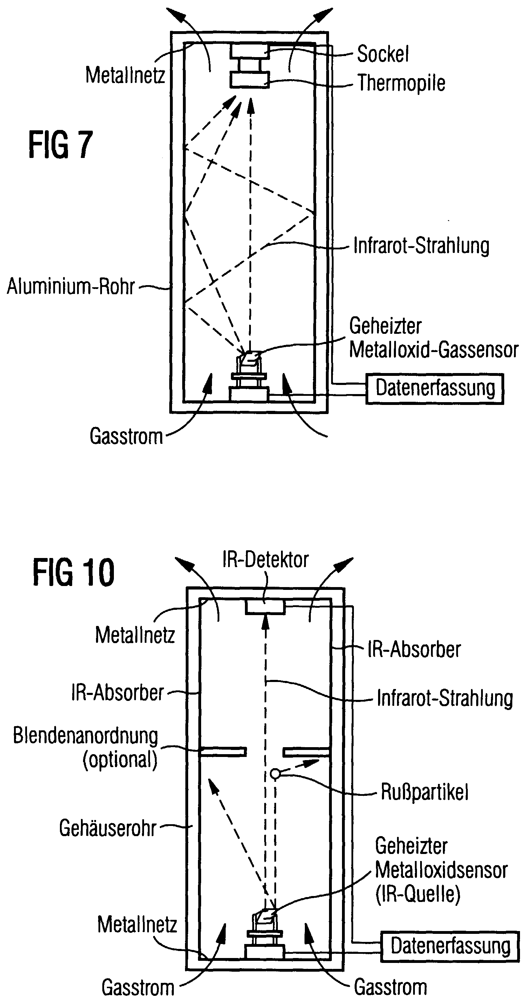

- Figur 7: zeigt eine erfindungsgemäße Anordnung des Kombinationssensors,
- Figur 8: zeigt das gute Ansprechverhalten von Metalloxid-Gassensoren auf einen typischen Schwelbrand, wobei beispielsweise unmodifiziertes Ga₂O₃ und mit SiO₂ beschichtetes Ga₂O₃ für einen selektiven Wasserstoffsensor eingesetzt wird und einen Vergleich mit herkömmlichen Detektionsprinzipien,
- Figur 9: zeigt ein schlechtes Ansprechverhalten von Metal loxid-Gassensoren auf einen typischen offenen Brand, beispielsweise bei der Verwendung von unmodi fiziertem Ga₂O₃ und mit SiO 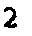 beschichtetem Ga 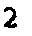 O 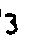 , beispielsweise einem Wasserstoffsensor, wobei ein Vergleich mit herkömmlichen Detektionsprinzipien angestellt wird,
- Figur 10: zeigt den Aufbau eines kombinierten Sensorsystemes bei dem direkt die Verminderung der Infrarotstrah lungsintensität durch das Auftreten von Rauch ge messen wird.

Die Figuren 1 bis 3 betreffen Erläuterungen zum Stand der Technik.

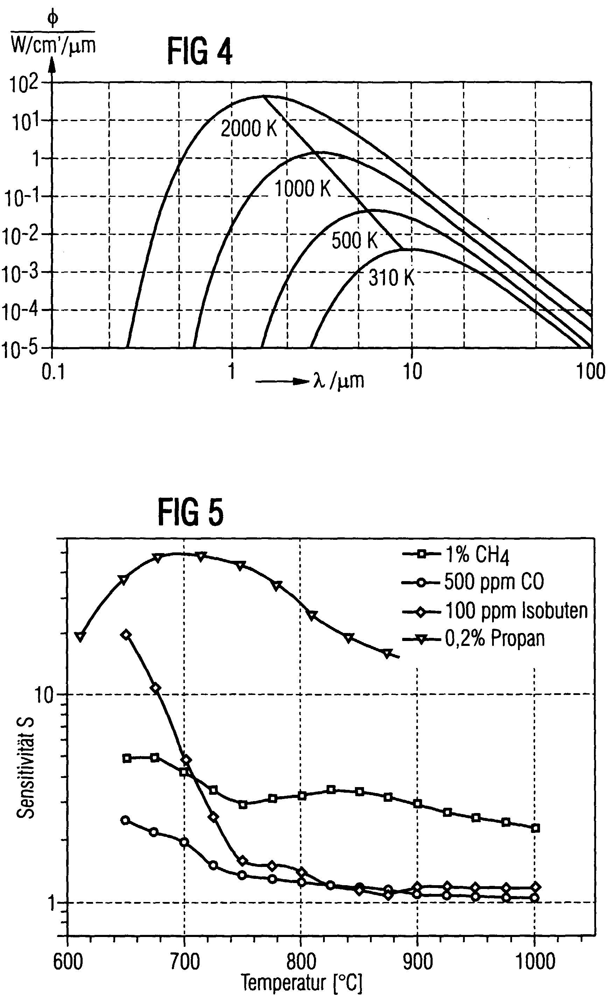

Die Figur 4 veranschaulicht, daß eine hohe spektrale Dichte bei einem beheizten Metalloxid-Gassensor vorliegt.

Vorteilhafterweise wird der Teil des Kombinationsgassensors, der als Strahlungsquelle dient, mit einer Heizungsregelung betrieben. Dies bewirkt, daß dieser Gassensor unbeeinflußt von Einwirkungen der Umgebung auf einer gleichen Temperatur betrieben wird. Im nicht geregelten Betriebsmodus könnte es andernfalls zu einer Temperaturänderung kommen, beispielsweise auch durch katalytische Verbrennung bestimmter Gase an der Senosoroberfläche. Dies würde eine Änderung der spektralen Dichte hervorrufen. Eine Änderung der spektralen Dichte bewirkt letztlich, eine Änderung der vom Strahlungsempfänger empfangenen Leistung, die als Änderung der Gaskonzentration fehl interpretiert werden könnte. Somit ist der Einsatz einer Heizungsregelung sinnvoll, wenn derartige Fehlfunktionen durch eine entsprechend ausgelegte Ansteuer- und Auswerteelektronik nicht detektierbar sind.

Wird anstelle eines Hochtemperatur-Gassensors auf der Basis halbleitender Metalloxide beispielsweise eine andere Strahlungsquelle verwendet, so liegt ein Gassensor vor, der auf anderen Gasnachweismechanismen beruht und zur Aktivierung dieser Mechanismen in irgendeiner Form ganz oder partiell aufgeheizt wird. Solche Gassensoren können beispielsweise Pellistoren und Wärmeleitfähigkeitssensoren sein.

Als Strahlungsempfänger können beispielsweise Thermopiles (Verschaltung von Thermoelementen) verwendet werden, sowie andere Strahlungsempfänger wie z.B. Photodioden und Photowiderstände, die auf dem pyroelektrischen Effekt beruhen, wie beispielsweise Halbleiterdioden oder allgemein pyroelektrische Detektoren.

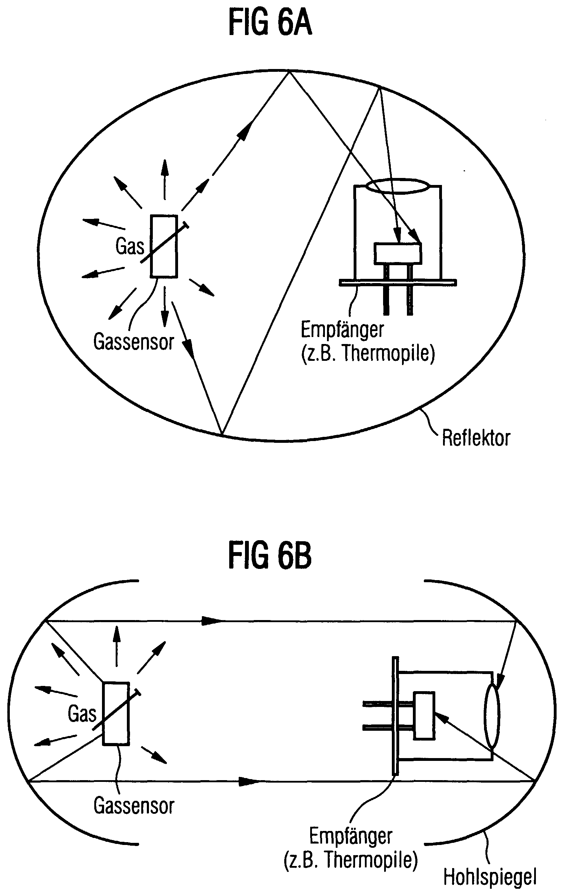

Vorteilhafterweise wird als Strahlungsquelle ein Gassensor eingesetzt, der nicht bei einer konstanten Temperatur betrieben wird, sondern abwechselnd bei zwei Temperaturen betrieben wird, so daß ein Temperaturwechsel stattfindet. Dieser Temperaturwechsel kann einerseits periodisch erfolgen oder andererseits nach Bedarf. Dieses Betriebsverfahren hat gegenüber dem statisch betriebenen Sensor mehrere Vorteile. Zum einen kann im Mittel die für die Sensoranordnung benötigte Heizleistung verringert werden. Bei geeigneter Wahl der Temperatur funktioniert der Strahler auch weiterhin als Gassensor und liefert auch in Zeiten reduzierten Leistungsbedarfes Meßwerte. Diese können bei Überschreitung gewisser Schwellwerte eine Messung bei höheren Temperaturen veranlassen, bei denen möglicherweise Strahlungsgassensor und optischer Gassensor exaktere und zuverlässigere Meßwerte liefern. Durch den Temperaturwechsel kann auch das Spektrum der Gase erweitert werden, die mit dem geheizten Gassensor detektiert werden können. Hierfür können die temperaturabhängigen Gassensititäten ausgenutzt werden. In Figur 5 werden die Verläufe der temperaturabhängigen Gassensitivitäten für verschiedene Gase eines elektrischen Leitfähigkeitssensors auf der Basis des halbleitenden Galliumoxides dargestellt.

Eine weitere Variante der Erfindung besteht darin, daß man durch periodisches Ausschalten der Strahlungsquelle den Einfluß der sog. Hintergrundstrahlung feststellen kann. Dies ist die Strahlung, die von anderen Körpern der Umgebung abgestrahlt und möglicherweise ebenfalls vom Detektor empfangen wird. Dadurch ist es möglich, den Nullpunkt beispielsweise eines Thermopiles festzustellen.

Durch die stetige Variation der Temperatur, die in Abwandlung zur vorgenannten Verfahrensweise durchaus nicht auf zwei diskrete Temperaturwerte eingeschränkt sein muß, ist auch eine Eichung der Temperatur des Hochtemperaturgassensors möglich. Hierbei werden die funktionalen Anhängigkeiten des elektrischen Widerstandes, beispielsweise eines als Strahlungssensor dienenden Metalloxid-Leitfähigkeitssensors einerseits und andererseits der spektralen Dichte des Strahlers bei einer festen Frequenz, die von einem Thermopile detektiert wird, ausgenutzt.

Fur die präzise Bestimmung der Strahlertemperatur wird bei Einsatz eines Gassensors auf der Basis halbleitender Metalloxide in diesem Strahlungssensor mit geringem technologischem Aufwand ein präziser Hochtemperatur-Meßfühler integriert. Dies ermöglicht eine genaue Regelung der Strahlertemperatur.

Eine besonders vorteilhafte Ausgestaltung besteht darin, die gassensitive Strahlungsquelle nicht notwendigerweise, wie oben beschrieben, auf andere Gase einzustellen. Sie kann auch dazu verwendet werden, um das gleiche Gas zu detektieren, wie die optische Sensoranordnung nach dem Absoptionsprinzip. Sie kann somit deren Meßergebnis auf Plausibilität überprüfen. Bei einem Einsatz der Sensoranrordnung, z.B. als Methan-Warnergerät können Fehlalarme vermieden werden. Es ist vorstellbar, daß eine Kondensatbildung auf einem Thermopile-Gehäuse durch plötzliche Zunahme der relativen Luftfeuchte in der umgebenden Atmosphäre sich nachteilig auswirken könnte. Ein solches Kondensat könnte die Strahlung zusätzlch absorbieren. Diese Abnahme der vom Thermopile empfangenen Strahlung könnte so interpretiert werden, als ob ein strahlungsabsorbierendes Gas vorhanden wäre. Die alleinige Auswertung des mit dem optischen Detektor erhaltenen Meßsignales würde somit zu einem Fehlalarm führen, wenn der Detektor z.B. als Methan-Warner eingesetzt werden wurde. Durch Verwendung der erfindungsgemäßen Anordnung ist es jedoch möglich, mit dem Kombisensor das Meßsignal des gassensitiven Strahlers mit dem des optischen Detektors zu vergleichen. Funktionsbedingt ist die Kondensatbildung bei Hochtemperatur-Gassensoren weitestgehend auszuschließen. Allerdings zeigen die Meßsignale der verschiedenen Meßprinzipien unterschiedlich stark ausgeprägte feuchte Empfindlichkeit. Bei einem Leitfähigkeits-Gassensor auf der Basis des halbleitenden Metalloxides Ga 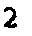 O 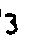 ist beispielsweise eine Änderung des als Meßsignal dienenden elektrischen Widerstandes des Sensors um ca. 20 bis 30% bei einem Feuchtigkeitswechsel festzustellen, wenn die Betriebstemperatur bei 800°C liegt. Demgegenüber verursacht eine zu detektierende Methan-Konzentration von 0,5% (10% LEL-Lower Explosive Limit) eine Widerstandsänderung um den Faktor ca. 3,5. Dieser Zusammenhang ist der Figur 5 zu entnehmen. Ein Alarm wird ausgelöst, wenn durch beide Sensorprinzipien das zu detektierende Gas in entsprechender Konzentration nachgewiesen wird.

Eine vorteilhafte Ausgestaltung der Erfindung sieht die Verbesserung der Absorption bei dem im Kombisensor enthaltenen Absorptionssensor vor, in dem das Produkt aus der Gaskonzentration mit der Länge des Lichtweges innerhalb des Zielgases maßgeblich vergrößert bzw. verbessert wird. Durch Verlängerung des optischen Weges ist es also möglich, geringere Konzentrationen eines Gases nachzuweisen. Der optische Weg kann beispielsweise mit geringem Aufwand unter Ausnutzung der Reflexion, entweder diffus oder spiegelnd an entsprechenden Flachen, im Vergleich zu einer direkten Einstrahlung von der Strahlungsquelle zum Strahlungsempfänger, verlängert werden. Entsprechende Ausgestaltungen werden durch die Figuren 6a und 6b wieder gegeben. Bei Verwendung einer solchen Anordnung ist es prinzipiell auch möglich, die spiegelnde Oberfläche derart zu beschichten, daß sie Wellenlängen selektiv reflektiert und zwar in dem Bereich der Strahlung, in dem die entsprechenden Absorptionslinien des zu detektierenden Gases liegen. In diesem Fall kann auf den wellenlängen-selektiven Filter im Gehäuse des Thermopiles verzichtet werden.

Durch Verwendung eines zweiten Thermopiles kann man in die erfindungsgemäße Anordnung ein passives Referenzelement einfügen. Dieses zweite Thermopile kann im gleichen Abstand wie das erste Thermopile vom geheizten Gassensor oder in einem davon abweichenden Abstand angeordnet werden. Ist das zweite Thermopile z.B. in einem hinreichend geringen Abstand vom Objekt angeordnet, so können durch die resultierende geringe Absorptionsstrecke keine Gase mehr detektiert werden. Änderungen des Ausgangssignales des zweiten Thermopiles sind dann ein Maß für eventuell auftretende Driften des Bauelementes oder auch für eine geänderte Hintergrundtemperatur, die sich im gleichen Maß auch beim ersten Thermopile wirksam niederschlagen hat. Der Einfluß solcher unerwünschten Effekte kann somit ausgeschlossen werden. Außerdem kann das zweite Thermopile bei geeigneter Wahl des zugehörigen Filters dazu verwendet werden, mit unveränderter Leitstungsaufnahme der Anordnung ein zweites Gas selektiv zu detektieren.

Die besonderen Vorteile eines erfindungsgemäßen Kombisensors bestehen darin, daß durch das Freihalten der optischen Fenster eine größere Langzeitstabilität des verwendeten Sensorsystemes gegeben ist. Die Kurzzeitstabilität des Meßsignales wird ebenfalls verbessert, wobei die erzielten Vorteile ohne Zusatzkosten erreicht werden können bzw. die Kosten für eine aufwendigere Auswerteelektronik eingespart werden. Der Kern liegt darin, daß in dem kostengünstigen Mehrnutzen der Sensoranordnung, der darin besteht, daß die grundsätzlich vorzusehende Strahlungsquelle selbst Gase detektieren kann. Hinzukommt die Möglichkeit von kostengünstigen Plausibilitätsprüfungen bei Gasmessungen und die Möglichkeit der exakten Strahlertemperatur-Messung unter Einstellung mit integrierten präzisen Hochtemperatur-Fühlern.

Ein erfindungsgemäßer Sensoraufbau kann beispielsweise dazu verwendet werden, um die Raumluft auf toxisches Kohlenmonoxid selektiv und auf explosive Gase, wie beispielsweise Erdgas und Flüssiggas breitbandig zu detektieren. Prinzipiell kann mit dem gleichen Aufbau auch Methan selektiv sowie ein anderes Gas, wie beispielsweise geruchsbelästigende Gase in Summe detektiert werden. Durch die Verwendung des erfindungsgemäßen Sensoraufbaues können alle genannten Anwendungen erfaßt werden. Die Kosten für den verwendeten Aufbau sind verglichen mit dem damit verbundenen Mehrnutzen geringfügig höher, als z.B. bei der ausschließlichen Detektion des selektiv zu messenden Gases.

Die Verwendung eines integrierten Aufbaues bestehend vorteilhafterweise aus einem Metalloxid-Halbleitersensor und einer Messung der Lichtstreuung durch Rauchpartikel löst ebenfalls die gestellte Aufgabe. Dabei wird der Metalloxid-Halbleitersensor als Infrarot-Lichtquelle für die Messung der Lichtabschwächhung bzw. Lichtstreuung verwendet. Die Erfindung zeichnet sich dadurch aus, daß durch den Metalloxid-Halbleitersensor Schwelbrände und durch die Messung der Lichtstreuung die Rauchentwicklung offener Brände vorzugsweise detektierbar sind.

Branddetektoren erkennen beispielsweise Schwelbrände, die dadurch gekennzeichnet sind, daß Emissionen von unverbrannten Gasen, wie beispielsweise Wasserstoff, Kohlenwasserstoff und Kohlenmonoxid vorliegen, jedoch recht geringe Rauchentwicklung damit verbunden ist. Offene Brände sind gekennzeichnet durch einen Luftüberschuß, der nur eine geringfügige Emission unverbrannter Gase bewirkt. Die offene Flamme bei Verbrennung typischer Materialien im Haushalt und im Büro ist jedoch von einer starken Rauchentwcklung begleitet.

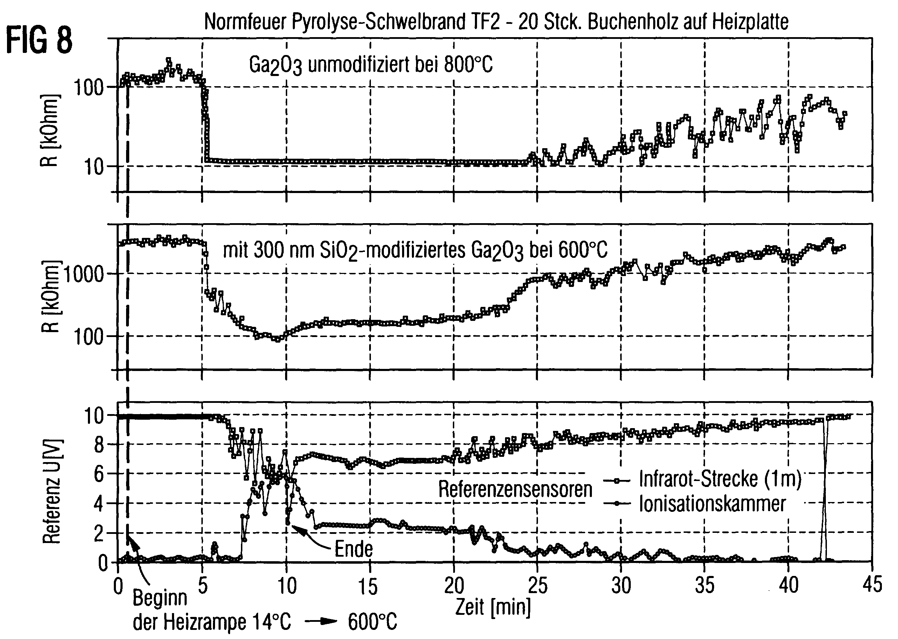

In Figur 8 sind im unteren Teil zusätzlich die Reaktionen zweier Referenzsensoren angegeben, die üblicherweise zur Branddetektion eingesetzt werden. Dabei wird die Reaktion einer Ionisationskammer und die einer Infrarot- Absorptionsstrecke als Vergleichswert herangezogen. Auch diese Referenzsensoren erkennen die Schwelbrände, jedoch mit starker Zeitverzögerung gegenüber den Metalloxidsensoren und unzureichendem Rückstellverhalten.

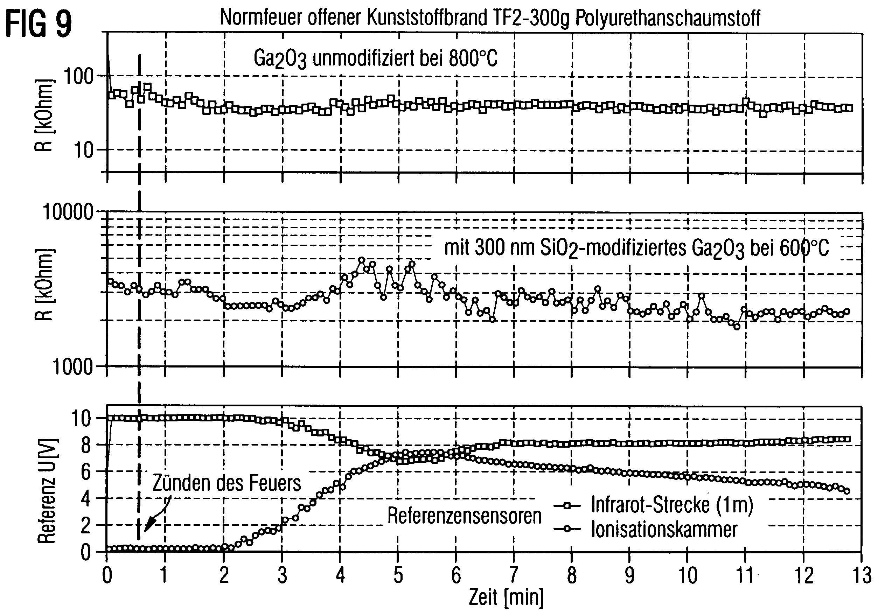

Es existieren bestimmte Formen offenber Brände, bei denen Metalloxidsensoren nur unzureichende Signale liefern, wie es in Figur 9 dargestellt wird. Somit ist die Hinzunahme eines weiteren Meßprinzipes notwendig. Bild 9 zeigt auch, daß die Infrarotmeßstrecke eine akzeptable Detektion gewährleistet. Daher wird erfindungsgemäß der Metalloxidsensor mit einer Infrarotmessung kombiniert, und zwar derart, daß die vom Metalloxidsensor ausgehende Infrarotstrahlung, die ein Probevolumen (Küvette, Absorptionsstrecke) durchläuft, wobei entweder die Verringerung der Transmission als Meßgröße(siehe Figur 10) oder das gestreute Licht direkt verwendet werden. Als Infrarotdetektor können z.B. eine Halbleiterdiode, eine Verschaltung von Thermoelementen (Thermopile) oder auch pyroelektrische Detektoren verwendet werden. Die Empfindlichkeit und Störsicherheit der Branddetektoren wird zusätzlich erhöht, wenn Infrarotdetektoren für einen optimalen Wellenlängenbereich verwendet werden. Bei Thermopiles und pyroelektrischen Detektoren ist für eine Anpassung für einen optimalen Wellenlängenbereich durch zusätzliche Bandpaßfilter, wie beispielsweise Interferenzfilter, üblich. Bei Halbleiterdioden geschieht dies durch Auswahl von deren Grundmaterial und ggf. zusätzlich durch einen zusätzlichen Bandpaßfilter. Damit wird die Anpassung an entsprechende Wellenlängenbereiche durchgeführt. Eine erfindungsgemäße Variante der Erfindung beinhaltet demnach:
- einen Metalloxidgassensor, der zusätzlich als Infrarotquelle genutzt wird,
- einen Infrarotdetektor, wie beispielsweise eine Halbleiterdiode, eine Verschaltung von Thermoelementen (Thermopile) oder auch einen pyroelektrischen Detektor,
- ein Gehäuse mit innenseitiger Infrarot absorbierender Beschichtung,
- optimal eine oder mehrere Blendenanordnungen, um die Wirkungsweise der Infrarot absorbierenden Beschichtung zu unterstützen, und
- eine Ansteuer- und Auswerteelektronik entsprechend Figur 10.

Das Detektionsverfahren zeichnet sich aus durch:
- extrem frühzeitiges Erkennen von Schwelbränden durch den Metalloxidsensor, bei gleichzeitig sichergestellter Erkennung offener Brände,
- Kostengünstigkeit (kostengünstige Komponenten), zweifache Nutzung des Metalloxidsensors),
- hohe Stabilität, da der temperaturgeregelte Metalloxidsensor eine äußerst konstante Infrarotquelle darstellt und
- hohe Störsicherheit.

Durch spezielle Betriebsverfahren (z. B. Temperaturzyklen des Metalloxidsensors) kann die einwandfreie Funktion des optischen Weges im Sinne eines Sensorselbsttestes erhöht werden, wodurch sich eine hohe wartungsfreie Betriebssicherheit realisieren läßt. Durch Vergleich der Sensorsignale der Einzeldetektoren kann erkannt werden, ob es sich um einen Schwelbrand oder einen offenen Brand handelt, was einem Überwachungssystem adäquat abgestufte Reaktionen ermöglicht.

## Patentansprüche

1. Kombinierter Gassensor zur Detektion von mindestens einem Gas oder von Partikeln oder einer Kombination daraus, bestehend aus:
- einem beheizten Gassensor,
- einem Gassensor, der nach der Infrarot-Absorptions-Analyse funktioniert mit einer Infrarotstrahlungsquelle, einer Absorptionsstrecke und einem Infrarotstrahlungs detektor,
wobei der beheizte Gassensor gleichzeitig die Infrarot-Strahlungsquelle für den Absorptionssensor darstellt.

2. Gassensor nach Anspruch 1, worin die Infrarot-Strahlungsquelle, die Absorptionsstrecke und der Infrarotstrahlungsdetektor in Reihe in einem senkrecht positionierten beidseitig offenen Rohr angeordnet sind und die Strahlungsquelle unten positoniert ist.

3. Gassensor nach Anspruch 1 oder 2, worin die Anordnung aus Infrarotstrahlungsquelle, Absorptionsstrecke und Infrarotstrahlungsdetektor zur Verlängerung der Absorptionsstrecke zumindest teilweise mit Reflektoren umgeben ist oder ein die Anordnung umgebendes Rohr innen reflektierend ausgebildet ist.

4. Gassensor nach Anspruch 1 oder 2, worin die Anordnung aus Gassensor, Absorptionsstrecke und Infrarotstrahlungsdetektor zumindest teilweise mit Infrarotlicht-absorbierenden Flächen umgeben ist oder ein die Anordnung umgebendes Rohr innen Infrarotstrahlung-absorbierend ausgebildet ist.

5. Gassensor nach einem der vorhergehenden Ansprüche, worin der beheizte Gassensor ein Metalloxid-Gassensor ist.

6. Gassensor nach Anspruch 5, worin als Metalloxid-Gassensor ein Leitfähigkeitssensor auf der Basis des halbleitenden Ga 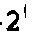 O 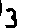 vorgesehen ist.

7. Gassensor nach einem der vorhergehenden Ansprüche, worin Infrarotstrahlungsdetektoren durch Auswahl des elekktronischen Bauelementes oder durch vorgeschaltete Bandpaßfilter auf einen vorbestimmten Wellenlängenbereich angepaßt sind.

8. Gassensor nach einem der vorhergehenden Ansprüche, worin der beheizte Gassensor eine Temperaturregelung aufweist.

9. Gassensor nach einem der vorhergehenden Ansprüche, worin der Infrarotstrahlungsdetektor ein Thermopile ist.

10. Gassensor nach Anspruch 9, worin ein zweites Thermopile als Referenzelement vorhanden ist.

11. Gassensor nach Anspruch 10, worin das Referenz-Thermopile im Gegensatz zum ersten Thermopile mit einer wesentlich kürzeren Absorptionsstrecke gekoppelt ist.

12. Gassensor nach einem der Ansprüche 3 oder 5 bis 10, worin die reflektierenden Flächen Wellenlängen-selektiv reflektieren.

13. Verfahren zum Betrieb eines Gassensors nach einem der Ansprüche 1 bis 12, worin
- an der beheizten Infrarotstrahlungsquelle gleichzeitig eine Gasdetektion durch eine Wärmeleitfähigkeitsmessung eines Gases oder die elektrische Leitfähigkeitsmessung eines in der Infrarotstrahlungsquelle vorhandenen Sensorelementes stattfindet,
- der Infrarot-Strahlungsdetektor die am Ende der Absorptionsstrecke entsprechend dem darin vorhandenen Gas vorliegenden Infrarotlichtanteile detektiert, und
- eine Steuer- und Auswerteelektronik die Detektionssignale verarbeitet.

14. Verfahren nach Anspruch 13, worin die Temperatur der Infrarotstrahlungsquelle variiert wird.

15. Verfahren nach Anspruch 14, worin die beheizte Infrarotstrahlungsquelle abwechselnd bei mindestens zwei unterschiedlichen Temperaturen betrieben wird.

16. Verfahren nach einem der Ansprüche 13 bis 15, worin die Infrarotstrahlungsquelle periodisch ausgeschaltet wird.

17. Verfahren nach einem der Ansprüche 13 bis 16, worin die beiden im Gassensor betriebenen Gasdetektionsverfahren auf dasselbe Gas eingestellt sind.

18. Verwendung eines Gassensors nach einem der Ansprüche 1 bis 12 zur selektiven Detektion von Kohlenmonoxid durch den Absorptionssensor und zur breitbandigen Detektion von explosiven Gasen durch den beheizten Sensor.

19. Verwendung eines Gassensors nach einem der Ansprüche 1 bis 12 zur selektiven Detektion von Kohlendioxid durch den Absorptionssensor verbunden mit der zusätzlichen Detektion von explosiven Gasen wie Methan oder Propan mit dem aufgeheizten Gassensor.

20. Verwendung eines Gassensors nach einem der Ansprüche 1 bis 12 zur Detektion von Methan durch den Absorptionssensor verbunden mit der Detektion von geruchsbelästigenden Gasen durch den aufgeheizten Sensor.

21. Verwendung eines Gassensors nach einem der Ansprüche 4 bis 12 zur Detektion von Rauchpartikeln, die durch offene Brände verursacht werden, im Absorptionssensor.

22. Verwendung nach Anspruch 21, worin zusätzlich durch die Detektion von Wasserstoff, Kohlenmonoxid oder Kohlenwasserstoffen Schwelbrände detektierbar sind.
